# EUROPEAN PATENT APPLICATION

(11) **EP 1 818 028 A2**
(43) Date of publication of application: **15.08.2007**
(21) Application number: 07101849.3
(22) Date of filing: 06.02.2007
(51) Int. Cl.: A61F 5/01, A43B 7/00, A43B 13/14

(54) **An improved postoperative footwear structure**

(30) Priority: 08.02.2006 IT TV20060004 U
(71) Applicant: Podarte S.r.l., 31044 Montebelluna TV (IT)
(72) Inventor: Buratto, Camillo, I-31044, Montebelluna (TV) (IT)
(74) Representative: Zanoli, Enrico

(57) **Abstract**

A postoperative footwear structure that comprises a relatively stiff rocker sole (2) and a plantar insole (3), which is operatively associated to said stiff rocker sole, so as to allow a comfortable heel walking of the user's foot.

## Description

The present invention relates to an improved postoperative footwear structure, particularly suitable for the treatment of diabetic plantar ulcers and postoperative rehabilitation following hallux valgus surgery. In further respects, the present invention relates also to an improved plantar insole and to an improved rocker sole for a postoperative footwear structure.

The footwear normally used in postoperative hallux valgus rehabilitation or for the treatment of diabetic ulcers on the fore- and mid-foot is generally provided with a stiff, rocker sole or with a sole suitable for heel-walking, i.e. a sole that is shaped so as to enable a type of walking, according to which the body weight is transferred from the fore-foot to the heel.

A first disadvantage of the postoperative footwear of the known type resides in that the patient is not prevented at all from returning in a short time to a normal footstep. Further, in the known post operative footwear, the tip of the user's foot naturally tends to lower and flex during the walking phase of pushing off the ground. This toe flexion often causes hyper-pressure on the fore-foot and there is often a tendency to bend the surgery wires, which may be present after some types of surgical operations, for example when a correction of the toe axis by insertion of a metal wire is provided.

A further disadvantage of the postoperative footwear of the known type consists in that the heel walking activity (i.e. the action of walking with the foot in a talipes calcaneus position) is generally difficult and crutches are often needed to maintain some stability. This is basically due to the fact that in a known piece of postoperative footwear the sole has a remarkable thickness at the rear-foot portion and a negligible thickness (almost equal to zero) at the fore-foot portion, so as to compel the user's foot to a heel walking activity. This remarkable difference of thickness between the rear-foot and the fore-foot portions of the sole notoriously determines considerable balancing problems to the user.

Therefore, it is an object of the present invention to provide a postoperative footwear structure, which allows the overcoming of the mentioned drawbacks.

A further object of the present invention is to provide a postoperative footwear structure, which has a high level of flexibility in use and which is more comfortable to the user, particularly during a heel walking activity.

Yet an additional object of the present invention is to provide, which may be simply realised at industrial level at competitive costs.

Thus, the present invention provides a footwear structure, according to the following claims 1-7.

In some further respects, the present invention provides also a plantar insole, according to the following claims 8-9, and a rocker sole, according to the following claims 10-12.

The footwear structure, according to the present invention, avoids the disadvantages described above, since it is provided with a combination of a special rocker sole and plantar, which enables the foot to rest on the heel while keeping the fore-foot substantially raised in a calcaneus talipes position, preventing at the same time from the elevation of the counter-lateral limb, which would cause inevitable postural damages.

The plantar insole, according to the present invention, allows to naturally placing the user's foot in a talipes calcaneus position, since it comprises portions having different thickness and/or different density or shore level.

The stiff rocker sole, according to the present invention, has a reduced height, since it is preferably provided with a stiff inner insert at the central portion. A greater flexibility of use is thereby possible, for example in association with a flat insole, with a flat pressure-relieving plantar, with a talipes calcaneus walking plantar.

Further features and advantages of the postoperative footwear stcucture, according to the present invention, will be described in more details hereinafter with particular reference to the attached drawings, wherein:
- Figure 1 shows a partially cut-way side view of the postoperative footwear, according to the present invention, in a preferred but not limiting embodiment; and
- Figure 2 shows a section view of a rocker sole, used in the post operative footwear of figure 1; and
- Figure 3 shows a perspective view of the plantar insole and of the containment ring, used in the post operative footwear of figure 1; and
- Figure 4 shows a planar view of the plantar insole, used in the post operative footwear of figure 1; and
- Figure 5 shows a side view of the plantar insole, used in the post operative footwear of figure 1; and
- Figure 6 shows a perspective view of the containment ring, used in the post operative footwear of figure 1.

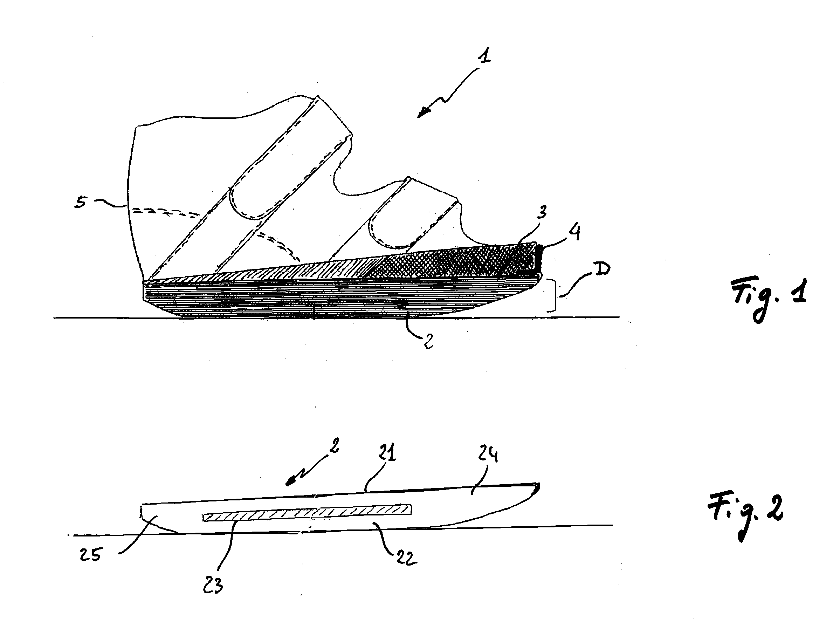

Referring now to the cited figures, the postoperative footwear 1, according to the present invention comprises a relatively stiff rocker sole 2, which may be associated to a suitable upper 5 for foot containment purposes. The rocker sole 2 is upwardly operatively associated with a plantar insole 3.

Preferably, the plantar insole 3 is releasably connectable with respect to the upper surface 21 of the rocker sole and it can be kept in position by insertion inside the upper 5 and/or by means of a suitable releasable connecting means (not shown), such as a Velcro^{™} strip.

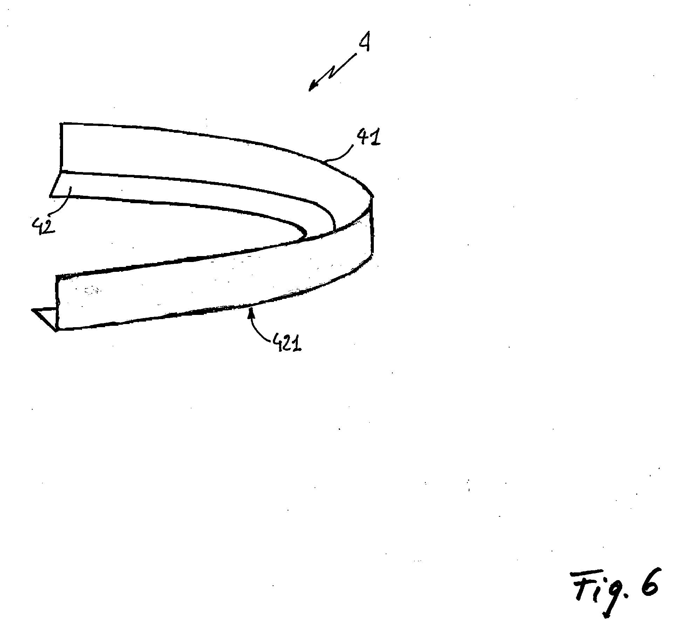

In a preferred embodiment, the present invention comprises also a removable anterior containment ring 4 that can be operatively associated to the rocker sole. The containment ring 4 (figure 6) preferably comprises an annular wall 41, substantially vertical, which is associated to an annular planar wall 42 that is placed in use substantially parallel to the upper surface 21 of the rocker sole 2. The ring 4 may be releasably connected at the tip of the rocker sole 2 at the lower surface 421 of the planar wall 42. The connection is preferably obtained by means of releasably connecting means (not shown), such as an annular Velcro^{™} strip (not shown).

The plantar insole 3 preferably comprises in fact a rear-foot portion 31 and a fore-foot portion 32. Preferably, the rear-foot portion 31 extends from the heel region up to behind the metatarsal heads of the user's foot while the fore-foot portion 32 is positioned at the metatarsal region of the user's foot.

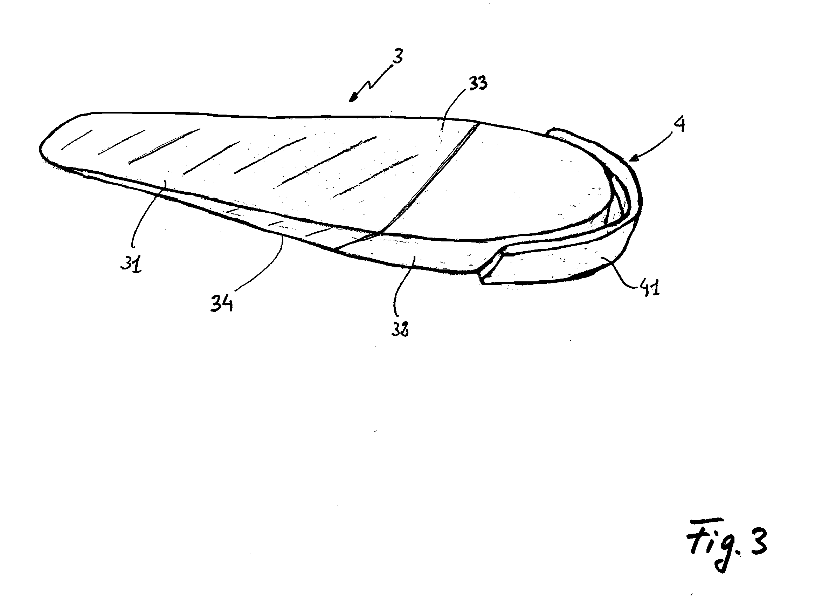

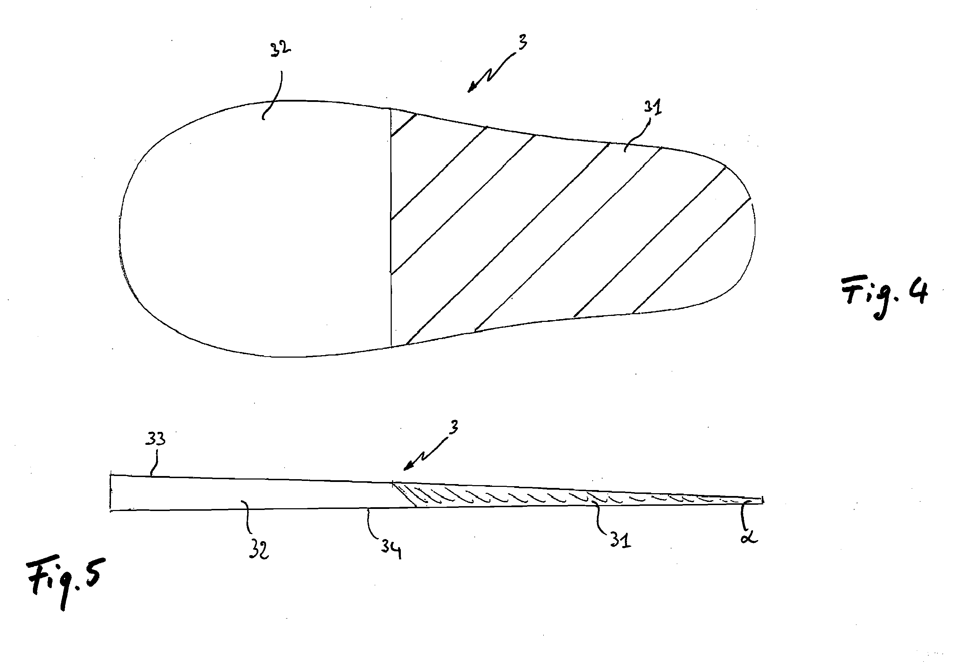

The plantar insole 3 is preferably provided with a wedge-shaped structure. To this aim, the rear-foot portion 31 is arranged with a lower thickness with respect to the fore-foot portion, so as to define an inclined upper surface 33 for the rest of the foot, which is thereby placed in a talipes calcaneus position. The angle α between the upper surface 33 and the lower surface 34 of the plantar insole 3 can be advantageously designed, so as maintain the user's foot in a comfortable rest portion. Preferably, the angle α varies in a range from 6° to 10° (figure 4).

The plantar insole 3 may be advantageously made of PU (Poly-Urethane) or EVA (Ethylene Vinyl Acetate) or other equivalent materials. Advantageously, the rear-foot portion 31 the plantar insole 3 is made of a harder or higher density material with respect to the material used for the fore-foot portion 32. In particular, the rear-foot portion 31 is made of a material with a shore in the range from 40 to 60 while the fore-foot portion 32 may be made of a material with a shore in the range from 25 to 35. In the practical realisation of the plantar insole 3, shores of 50 and 30 have been selected respectively for the rear-foot portion 31 and for the fore-foot portion 32.

In a preferred embodiment the rocker sole 2 is preferably made of PU o EVA as well, and it comprises at least an insert 23 made of a relatively stiff material, such as for example wood, carbon fibers, glass fibers, other composite materials, metal materials or plastic materials.

The insert 23 is preferably embedded in a central portion 22 of the sole 2, which extends approximately at the mid-foot region of the user's foot. The use of the insert 23 is quite advantageous, since it allows the reduction of the thickness of the central portion 22, which may be kept substantially constant and lower than 26 mm. In this manner, it is possible to achieve a suitable sole stiffness with a total height D of the rocker sole 2, which is remarkably less (e.g. 22 mm) than the typical height of the orthopaedic stiff-soled footwear or splints of the state of the art.

The rocker sole 2 is advantageously shaped to further improve the walking activity of the user. In particular, the tip portion 24 of the rocker sole 2 is upwardly curved, so as to help the completion of walking phase of pushing the foot off the ground. Similarly, the back portion 25 of the rocker sole 2 is smoothly upwardly curved, so as to facilitate the transition to the walking phase of pushing the foot off the ground.

It has been seen that the footwear structure 1, according to the present invention, allows to achieve the intended aims and objects.

A wedge-shaped plantar structure 3 enables the pressure to be displaced to the rear of the foot, thus relieving the operated or ulcerated fore-foot, and simultaneously resolving the described instability problems. In this manner a more comfortable heel walking activity is possible.

The plantar insole 3 with portions 31-32 having different density or shore level is particularly advantageous, since the greater density at the rear-foot portion 31 facilitates the foot to be placed in a talipes calcaneus position, relieving part of the pressure from the metatarsals, while a lower density at the fore-foot portion 32 helps to absorb shocks from the ground and it enables to achieve a self-modelling of the portion 32 to the shape of the user's foot.

The stiff insert 23 allows to reducing the total height of the rocker sole 2 while ensuring a suitable stiffness, thereby avoiding postural problems during the walking activity, which may affect the knee joints, the hip joints and the vertebral column. Thus, the rocker sole 2 can be suitably associated different kinds of sole, with a remarkable flexibility of use. It is possible to choose between an orthopaedic option with a common flat plantar insole (not shown), which relieves the affected metatarsals, and the plantar insole 3, which transfers the load to the back of the foot, all without any instability problems.

The combined presence of the stiff rocker sole 2 and the wedge-shaped plantar 3 enables heel walking while immobilising the mid- and fore-foot joints. At the same time, it is possible to prevent from the downloading of stress on the metatarsal phalangeal joint and to transfer body weight from the fore-foot to the back of the foot, which enables a correct and comfortable heel walking.

The use of the containment ring 4 is particularly advantageous, since it allows the improving the flexibility of use of the footwear structure 1. In fact, without the ring, the surface, on which the foot rests is substantially flat, including the anterior portion, and it does not block any metal wires protruding as a result of surgical interventions. Using the ring 4, the rocker sole 2 is provided with a containing structure, which enables the insertion of one or more plantar insoles, such as the plantar insole 3, which are quite useful to relieve pressure on the metatarsals or ulcers.

The footwear structure, according to the present invention, is characterised by a simple structure, which allows an easy manufacture at industrial level, at competitive costs.

## Claims

1. A postoperative footwear structure **characterised in that** it comprises a relatively stiff rocker sole and a plantar insole, which is operatively associated to said stiff rocker sole, so as to allow a comfortable heel walking of the user's foot.

2. A postoperative footwear structure, according to claim 1, **characterised in that** said plantar insole comprises a rear-foot portion and a fore-foot portion, said rear-foot portion being arranged with a lower thickness with respect to said fore-foot portion, so as to place the foot in a talipes calcaneus position.

3. A postoperative footwear structure, according to claim 2, **characterised in that** the rear-foot portion of said plantar insole is made of a harder material with respect to the material used for the fore-foot portion of said plantar insole.

4. A postoperative footwear structure, according to one or more of the previous claims **characterised in that** said rocker sole is provided with one or more inserts made of a relatively stiff material.

5. A postoperative footwear structure, according to one or more of the previous claims **characterised in that** said rocker sole comprises a central portion which has an approximately constant thickness.

6. A postoperative footwear structure, according to claim 5, **characterised in that** said a central portion has a thickness lower than 26 mm.

7. A postoperative footwear structure, according to one or more of the previous claims **characterised in that** it comprises a removable anterior containment ring that can be operatively associated to said rocker sole.

8. A plantar insole for a postoperative footwear structure **characterised in that** it comprises a rear-foot portion and a fore-foot portion, said rear-foot portion being arranged with a lower thickness with respect to said fore-foot portion, so as to place the foot in a talipes calcaneus position.

9. A plantar insole for a postoperative footwear structure **characterised in that** it comprises a rear-foot portion and a fore-foot portion, said rear-foot portion being made of a harder material with respect to the material used for said fore-foot portion.

10. A rocker sole for a postoperative footwear structure **characterised in that** it comprises a central portion, which has an approximately constant thickness.

11. A rocker sole, according to claim 10, **characterised in that** said central portion has a thickness lower than 26 mm.

12. A rocker sole **characterised in that** said central portion comprises with one or more inserts made of a relatively stiff material.
